# EUROPEAN PATENT APPLICATION

(11) **EP 2 604 680 A1**
(43) Date of publication of application: **19.06.2013**
(21) Application number: 10855859.4
(22) Date of filing: 12.08.2010
(51) Int. Cl.: C12M 3/00

(54) **AUTOMATIC CULTURE DEVICE**

(71) Applicant: Hitachi, Ltd., Chiyoda-ku Tokyo 100-8280 (JP)
(72) Inventor: NOZAKI, Takayuki, 6-1, Marunouchi 1-chome Chiyoda-ku, Tokyo 100-8220 (JP); KOBAYASHI, Toyoshige, 6-1, Marunouchi 1-chome Chiyoda-ku, Tokyo 100-8220 (JP); MATSUOKA, Shizu, 6-1, Marunouchi 1-chome Chiyoda-ku, Tokyo 100-8220 (JP); NAKAJIMA, Ryota, 6-1, Marunouchi 1-chome Chiyoda-ku, Tokyo 100-8220 (JP)
(74) Representative: MERH-IP Matias Erny Reichl Hoffmann
(86) International application number: PCT/JP2010/005056
(87) International publication number: WO 2012/020458

(57) **Abstract**

In order to uniformly sow cells on the culture surface of a cartridge-type closed culture vessel and remove bubbles contaminating in liquid culture medium in the course of automatic culture, an automatic culture equipment is provided with a flow-controlling mechanism section for solving the non-uniformity in cell distribution by, after filling up a culture space in the cartridge-type closed culture vessel, said cartridge-type closed culture vessel being in an upright position, with cell suspension, turning the cartridge-type closed culture vessel into a horizontal position, and then repeatedly supplying the cell suspension and sucking the same multiple times to thereby create a mixing flow in the cell suspension. In this process, the liquid in channels is efficiently sent by applying a reduced pressure and an elevated pressure respectively to a channel on one side and a channel on the opposite side, using two syringes and check valves connected to the channels, to thereby load forces to the liquid from both sides. The liquid can be sent without any cell loss by conducting the same operations of the flow-controlling mechanism section in a tank and in a cell bag.

## Description

### Technical Field

The present invention relates to an automatic culture equipment that uses a cartridge-type closed culture vessel to culture cells or tissues by automatic manipulation, and particularly, to an automatic culture equipment which is capable of fabricating regenerated tissues which may be used in a regenerative medical treatment.

### Background Art

A regenerated tissue which is transplanted as a regenerative medical treatment is manufactured based on GMP (good manufacturing practice) guidelines which is standard of manufacturing and quality management of drugs and medicines. Generally, the regenerated tissue is fabricated by an expert having a specialized cell culture technique in a CPC (cell-processing center) that provides a clean manufacturing environment in accordance with an SOP (standard operating procedure). As the GMP guidelines, provisions such as Ministry of Health and Welfare Ordinance No. 179 or Pharmaceutical Department Document No. 480 which are established by Ministry of Health and Welfare have already come in force in Japan. Outside of Japan, the GMP guidelines are issued mainly by, for example, organizations in Europe and the United States such as America Food and Drug Administration or European Commission.

The regenerated tissue is manufactured by an expert having a specialized cell culture technique in an environment where the safety and cleanness established by such a provision are managed. Therefore, considerable labor costs, labor, and operational costs are required. Further, since whole manufacturing processes are manually performed, there is limitation in the amount of regenerated tissues to be manufactured. As a result, the manufacturing cost of manufacturing the regenerated tissue increases, which prevents the regenerative medical treatment from being spread. Therefore, in order to conquer such a current situation, it is required to introduce an automatic culture equipment that can automate some or all of the culture processes. The culture processes is performed not by manual labor, but by the automatic culture equipment, so that saving in labor cost, reduction in cost and mass production are achieved. In addition, the manipulation by the automatic culture equipment is so stable that contribution to the uniformity in the quality of the manufactured regenerated tissues is also expected. Here, even though the automatic culture equipment cultures a cell instead of being performed manually, it is considered that the automatic culture equipment necessarily satisfies the GMP guidelines as in the manufacturing process by the manual operation. That is, based on a scientific basis, the automatic culture equipment should show to manufacture a high quality regenerative tissue with a good reproducibility while maintaining the cleanliness.

A culture vessel which is used in the automatic culture equipment is mainly classified into an open culture vessel which is easily open/closed and has a large contact area with the outside, for example, a culture vessel having a lid and a closed culture vessel which is not easy to open/close and has a small contact area with the outside, for example, a cartridge type culture vessel. A culture technology that uses the open culture vessel has been already established and includes from a research and development stage to a manufacturing and selling stage of the regenerative medicine. Further, the culture technology that uses the open culture vessel is generally used for the culture by manual operation. However, the open culture vessel has a structure in which a culture medium easily overflows or spills and has a large contact area with the outside which causes a biological contamination. A specialized culture technology is required to culture a cell while maintaining sterility. An automatic culture equipment having a driving equipment such as a robot arm or a conveying robot which is suitable for the open culture vessel has been already reported.

In the meantime, the closed culture vessel is positioned as a next generation culture vessel that is assumed to be applied in an automatic culture equipment and is being developed in various research organizations. Even though a culture technology that uses the closed culture vessel and the automatic culture equipment is not sufficiently established, there have been some researches (see Patent Literature 1 and 2). Generally, the closed culture vessel has a structure in which the culture medium does not easily overflow and has a small contact area with the outside. Therefore, the closed culture vessel has an advantage in that the sterility is easily maintained as compared with the open culture one. As an example of the automatic culture equipment that uses the closed culture vessel, an automatic culture equipment, which uses a cartridge type closed culture vessel and connects the cartridge type closed culture vessel to the automatic culture equipment through a joint using a pressure bonding method to seed cells or change culture medium, has been reported (see Patent Literature 3 and 4). The above-mentioned cartridge type closed culture vessel has a valve structure and flows in/out the culture medium from/to the outside only through the valve structure. Further, inside of the cartridge type closed culture vessel exchanges gas, which is required to culture such as O₂ or CO₂, with the outside through a gas-permeable membrane. In addition, an automatic culture equipment that uses a closed culture vessel is disclosed in Patent Literature 5 and 6.

### Citation List

### Patent Literature

Patent Literature 1: Japanese Patent Application Laid-Open Publication No. 2008-113600
Patent Literature 2: Japanese Patent Application Laid-Open Publication No. 2004-089138
Patent Literature 3: Japanese Patent Application Laid-Open Publication No. 2006-320304
Patent Literature 4: Japanese Patent Application Laid-Open Publication No. 2006-149237
Patent Literature 5: Japanese Patent Application Laid-Open Publication No. 2006-141328
Patent Literature 6: Japanese Patent Application Laid-Open Publication No. 2004-208665

### Summary of Invention

### Technical Problem

The automatic culture equipment needs to satisfy the GMP guidelines as described above. Especially, it is required to maintain sterility in the culture environment. During the culture period, it is required to prevent bacteria from being invaded in a flow channel which may be directly brought into contact with cells through culture medium. Further, in order to stably perform regenerative medical treatment, uniform regenerated tissues are needed to be manufactured. As one of the conditions therefor, it is required to uniformly seed cells on a culture surface at the time of starting the cultivation. In order to satisfy the condition, when a cell suspension is sucked, it is required to avoid non-uniformity in the cell distribution caused by the sedimentation of the cells and perform the culture in the state of uniformity in the cell distribution. Further, if cells are seeded in the cell suspension with bubbles mixed therein, the cell distribution is non-uniformized due to a surface tension suspension. In other words, it is required to remove the bubbles from the cell suspension. Finally, for example, in order to manufacture a regenerated tissue which is used to regenerate cornea, even though the number of cells at the time of starting the culture is smaller than a large regenerated tissue such as skin, it is required to efficiently culture a small amount of cells in the automatic culture equipment, efficiently suck the full amount of cells from a cell bag, and prevent cell loss from components of a flow channel through which the cell is passes until the cells are seeded.

As an automatic culture equipment that uses a closed culture vessel, in Patent Literature 5, in order to use the closed culture vessel having a gas phase therein, it is possible to uniformly distribute the cells only by inclining the closed culture vessel multiple times at the time of seeding the cells. In the method, when the cells are cultured in a closed culture vessel only formed of a liquid phase such as, for example, a cartridge type closed culture vessel, since the liquid hardly moves even though the vessel is declined, the cells are not uniformly distributed. Further, in the case of Patent Literature 5, an air bubble, which is mixed in the flow passage in some reasons, may be removed from the gas phase part of the closed culture vessel. In the meantime, since the cartridge type closed culture vessel does not have the gas phase, if minute bubbles are mixed therein, it is difficult to remove the minute bubbles mixed. In Patent Literature 6 which is another Patent Literature which discloses the automatic culture equipment using the closed culture vessel, a seeding unit that is capable of diluting the cell suspension is provided between the culture vessel and a storing unit of a culture medium. However, since the automatic culture equipment aims to be used in a space, the flow passage may perform only a minimum function required to culture. Therefore, according to the method disclosed in Patent Literature 6, it is difficult to uniformize the cell distribution and remove the mixed bubbles.

As described above, in order to perform good regenerative medical treatment using an automatic culture equipment that cultures cells or tissues using a closed culture vessel, it is required to manufacture a uniform regenerated tissue. For this reason, it is necessary for cells to be uniformly seeded on a culture surface at the time of starting cultivation. Further, the culture needs to be performed in a state where there is no bubble in the closed culture vessel. However, bubbles may be mixed therein at the time of seeding cells, changing culture medium, and flowing various solutions. When the bubbles are mixed, a function that removes the bubbles is required. In addition, when a small amount of cells are cultured, especially, it is required to efficiently transfer the cell suspension and seed the cells without cell loss so as to culture all cells included in the cell suspension, which is injected into the cell bag, on the culture surface.

An object of the present invention is to provide an automatic culture equipment that is capable of uniformly seeding cells on a culture surface of a closed culture vessel and culturing the cells without generating bubbles in the closed culture vessel.

### Solution to Problem

In order to attain the above object, the present invention provides an automatic culture equipment that uses a cartridge type closed culture vessel having a culture space or room therein. The automatic culture equipment includes a first flow channel which is connected around one end of the cartridge type closed culture vessel and supplies a fluid in the culture space; a second flow channel which is connected around the other end of the cartridge type closed culture vessel and ejects fluid from the culture space; and fluid flow controlling mechanism section that is connected to the first and second flow channels and controls the fluid to be flowed to the cartridge type closed culture vessel. The fluid flow controlling mechanism section repeatedly supplies and sucks a small amount of cell suspension to the first flow channel in a state where the inside of the cartridge type closed culture vessel is full of the fluid cell suspension to control to create an agitation flow in the cell suspension in the cartridge type closed culture vessel.

Further, in order to attain the above object, the present invention provides an automatic culture equipment that uses a cartridge type closed culture vessel having a culture space therein. The automatic culture equipment includes a cell bag in which cell suspension is maintained; a culture medium bag in which a culture medium is received; a tank which is disposed between the cartridge type closed culture vessel and the cell bag to temporally reserve the cell suspension; a first flow channel which is provided above the tank and connects the cell bag, the culture medium bag, and the tank; a second flow channel that ejects gas in the tank; a third flow channel that is provided below the tank and connects the cartridge type closed culture vessel and the tank; a valve unit that opens and closes the first flow channel, the second flow channel, and the third flow channel; and a fluid movement controlling mechanism section that controls to supply the cell suspension and the culture medium to the tank and dilute the cell suspension. The controlling mechanism section controls to eject the bubbles in the cell suspension in the tank to the second flow channel and remove the bubbles from the cell suspension in the tank.

In addition, in order to attain the above object, the present invention provides an automatic culture equipment that uses a cartridge type closed culture vessel having a culture space therein. The automatic culture equipment includes a cell bag in which a cell suspension is maintained; a flow channel which is connected to the cell bag; and a flow movement controlling mechanism section that controls to move the cell suspension in the cartridge type closed culture vessel through the flow channel. The flow movement controlling mechanism section controls to apply a reduced pressure in the flow channel, change the pressure with respect to the cell suspension in the cell bag and move the cell suspension to the flow channel side, and repeatedly transfer a smaller amount of the cell suspension than the amount of the cell suspension in the cell bag and inject the transferred cell suspension in the cell bag when the cell suspension is transferred to the cartridge type closed culture vessel or the tank, to create an agitation flow in the cell suspension in the cell bag.

That is, the automatic culture equipment that uses a cartridge type closed culture vessel having a culture space therein according to an exemplary embodiment of the preset invention accomplishing the aforementioned object includes a flow channel that supplies gas and/or liquid, that is, a fluid to the culture space and a flow channel that ejects the fluid to the culture space. Further, each of the flow channels is connected to a syringe. The two syringes move in a synchronized state by a unit such as a pump. In addition, a check valve or a clack valve that limits the flow of air in one direction is connected between each of the syringes and the valve. When the two syringes are operated, an elevated pressure is applied to one of the flow channels which are connected to the cartridge type closed culture vessel and an elevated pressure is applied to the other flow channel, to change the pressure of the liquid which fills the cartridge type closed culture vessel from both sides to move the liquid. A pressure is applied so as to switch the elevated pressure state and the reduced pressure state to move the liquid even in a reverse direction.

Furthermore, according to an exemplary embodiment of the present invention, when the cell suspension is seeded in the culture space of the cartridge type closed culture vessel, a controlling mechanism that performs the following operations is provided. That is, when the cell suspension is flowed, the cell suspension is filled in the cartridge type closed culture vessel in a state where the cartridge type closed culture vessel is in an upright position so as to dispose the flow channel that ejects the fluid of the cartridge type closed culture vessel at an upper side and the flow channel that supplies the fluid of the cartridge type closed culture vessel at a lower side. Further, after filling up the inside of the cartridge type closed culture vessel with the cell suspension, the cartridge type closed culture vessel is turned into a horizontal position. In this state, a small amount of cell suspension is further supplied and the same amount of cell suspension is sucked again. The cell suspension is repeatedly supplied and sucked multiple times to create an agitation flow in the cell suspension in the cartridge type closed culture vessel. Further, the non-uniform state of the cell due to the sedimentation caused by the weight of the cell when the cell suspension is injected in a state where the cartridge type closed culture vessel is in a upright position is resolved. In a culture space in the cartridge type closed culture vessel, a diffusing machine, which is capable of speeding up to uniformly seed the cell in the culture space by diffusing the cell when the cell suspension flows in the culture space, is provided.

Further, the automatic culture equipment according to an exemplary embodiment of the present invention includes a tank, which dilutes the cell suspension, between the cartridge type closed culture vessel and a cell bag in which the cell suspension is maintained. This tank has a heater which serves as a heating section that may heat up the cell suspension to an appropriate temperature for the culture, for example, up to 37°C and maintain the temperature. The tank has a function of adjusting a concentration of the cell suspension by supplying the culture medium from the culture medium bag to the tank. Above the tank, a first flow channel, which connects the cell bag, in which the cell suspension is received, to the tank and a second flow channel that ejects an air in the tank, are provided. Below the tank, a flow channel that connects the cartridge type closed culture vessel to the tank is provided. An electronic valve, which configures a valve unit that may close the first flow channel, the second flow channel, and the third flow channel, is provided. With these configurations, a controlling mechanism, that receives cell suspension containing bubbles mixed by predetermined reasons in the tank and ejects the contained bubbles from the second flow channel to remove the bubbles from the cell suspension when the cell suspension is transferred from the cell bag, in which the cell suspension is received, to the tank, is provided.

In addition, the automatic culture equipment according to an exemplary embodiment of the present invention includes a controlling mechanism that sucks the air from the second flow channel while the first and third flow channels are closed by the electronic valve to reduce a pressure in the tank and maintain the state and removes the minute bubbles generated on the liquid surface of the cell suspension and then gradually returns the pressure in the tank to a normal pressure.

Furthermore, the automatic culture equipment according to an exemplary embodiment of the present invention includes a controlling mechanism that flows a small amount of cell suspension collected in the tank when the cell suspension is flowed to the cartridge type closed culture vessel, re-injects the transferred cell suspension in the tank, and repeatedly transfers and injects the cell suspension in the tank multiple times to create an agitation flow in the cell suspension in the tank and remove the non-uniform state of the cell due to the sedimentation caused by the weight of the cell when the cell suspension is collected in the tank. A driving unit of the liquid which is used herein is to the same as the unit for the cartridge described above.

Finally, according to the automatic culture equipment according to an exemplary embodiment of the present invention, the cell bag of the automatic culture equipment includes a controlling mechanism that flows a small amount of cell suspension in the cell bag when the cell suspension is transferred to the tank and re-injects the transferred cell suspension in the tank, and repeatedly transfers and injects the cell suspension in the tank multiple times to create an agitation flow in the cell suspension in the cell bag and remove the non-uniform state of the cell due to the sedimentation caused by the weight of the cell when the cell suspension is collected in the cell bag. A driving unit of the liquid which is used herein is the same as the unit for the cartridge described above.

### Advantages Effects of Invention

According to the automatic culture equipment according to the present invention, it is possible to flow the cell suspension and seed the cell while uniformly distributing the cells. Further, bubbles which are a cause of the non-uniformity of the cell distribution are removed. Therefore, it is possible to manufacture uniform regenerative tissue.

### Brief Description of Drawings

[FIG. 1] FIG. 1 is an overall configuration view of an automatic culture equipment with a flow channel mounted therein according to a first embodiment.
[FIG. 2] FIG. 2 is a view illustrating an overall configuration of the automatic culture equipment from which the flow channel is separated according to the first embodiment.
[FIG. 3A] FIG. 3A is a view illustrating an overall image of the flow channel which is provided in the automatic culture equipment according to the first embodiment.
[FIG. 3B] FIG. 3B is a view illustrating a base set that holds a part of the flow channel of the automatic culture equipment, which is related to a culture process, according to the first embodiment.
[FIG. 3C] FIG. 3C is a view illustrating a base set that holds a part of the flow channel of the automatic culture equipment, which is related to a culture process, according to the first embodiment.
[FIG. 4] FIG. 4 is a view illustrating a configuration of a cartridge type closed culture vessel according to the first embodiment.
[FIG. 5] FIG. 5 is a view illustrating a state where a diffusing machine, which diffuses the cell, is provided in a culture space of the cartridge type closed culture vessel, according to a modification embodiment of the first embodiment.
[FIG. 6] FIG. 6 is a view illustrating a whole flow channel when two cartridge type closed culture vessels are cultured according to the first embodiment.
[FIG. 7A] FIG. 7A is a view illustrating a flow (1) of a solution and an air in accordance with the movement of a syringe when the solution is transferred from the tank to the cartridge type closed culture vessel, according to the first embodiment.
[FIG. 7B] FIG. 7B is a view illustrating a flow (2) of a solution and an air in accordance with the movement of a syringe when the solution is transferred from the tank to the cartridge type closed culture vessel, according to the first embodiment.
[FIG. 8] FIG. 8 is a view illustrating a series of protocol, which cultures a cell using the automatic culture equipment, according to the first embodiment.
[FIG. 9A] FIG. 9A is a view illustrating an overview (1) of a protocol, which is performed by the flow channel in the tank, according to the first embodiment.
[FIG. 9B] FIG. 9B is a view illustrating an overview (2) of a protocol, which is performed by the flow channel in the tank, according to the first embodiment.
[FIG. 9C] FIG. 9C is a view illustrating an overview (3) of a protocol, which is performed by the flow channel in the tank, according to the first embodiment.
[FIG. 9D] FIG. 9D is a view illustrating an overview (4) of a protocol, which is performed by the flow channel in the tank, according to the first embodiment.
[FIG. 9E] FIG. 9E is a view illustrating an example of a structure, in which a heater is attached on the tank of the flow channel, according to the first embodiment.
[FIG. 10A] FIG. 10A is a view illustrating an overview (1) of a protocol, which is performed by the flow channel in the cartridge type closed culture vessel, according to the first embodiment.
[FIG. 10B] FIG. 10B is a view illustrating an overview (2) of a protocol, which is performed by the flow channel in the cartridge type closed culture vessel, according to the first embodiment.
[FIG. 10C] FIG. 10C is a view illustrating an overview (3) of a protocol, which is performed by the flow channel in the cartridge type closed culture vessel, according to the first embodiment.
[FIG. 10D] FIG. 10D is a view illustrating an overview (4) of a protocol, which is performed by the flow channel in the cartridge type closed culture vessel, according to the first embodiment.

### Description of Embodiments

Hereinafter, embodiments of an automatic culture equipment according to the present invention will be described in detail with reference to the drawings. Further, in the specification, gas or liquid or gas and liquid that flow in the flow channel of the automatic culture equipment may be collectively referred to as fluid. In addition, a mechanism such as a syringe or a syringe pump (both are referred to as a "syringe") or a rotation controlling unit, that is a rotation mechanism that rotates the cartridge type closed culture vessel, which move the fluid in the flow channel are collectively referred to as a fluid movement controlling mechanism section.

### First Embodiment

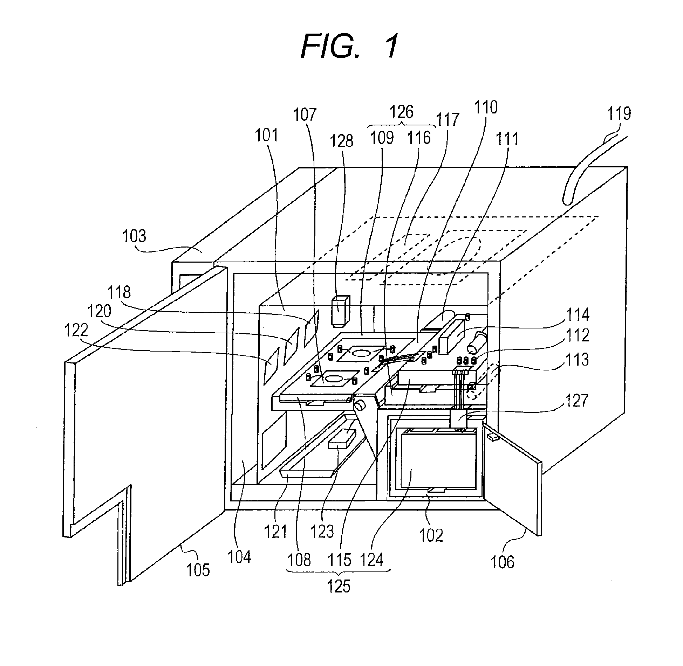

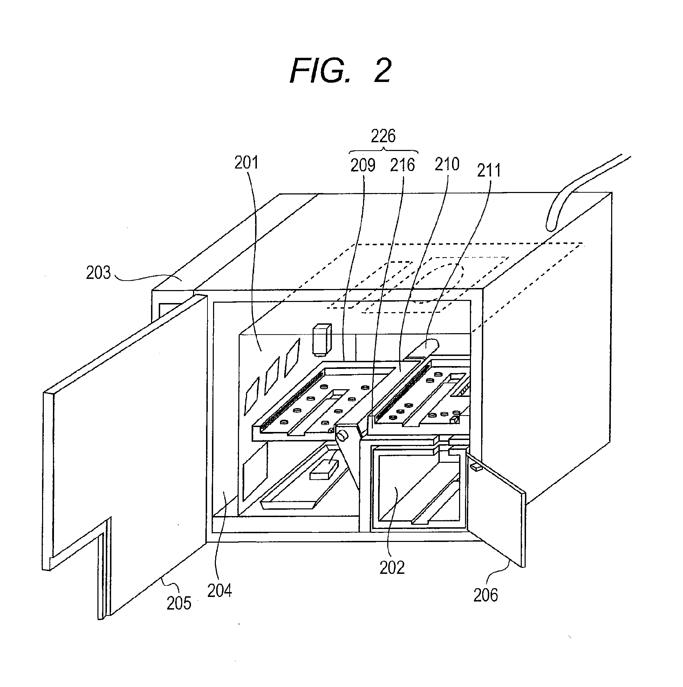

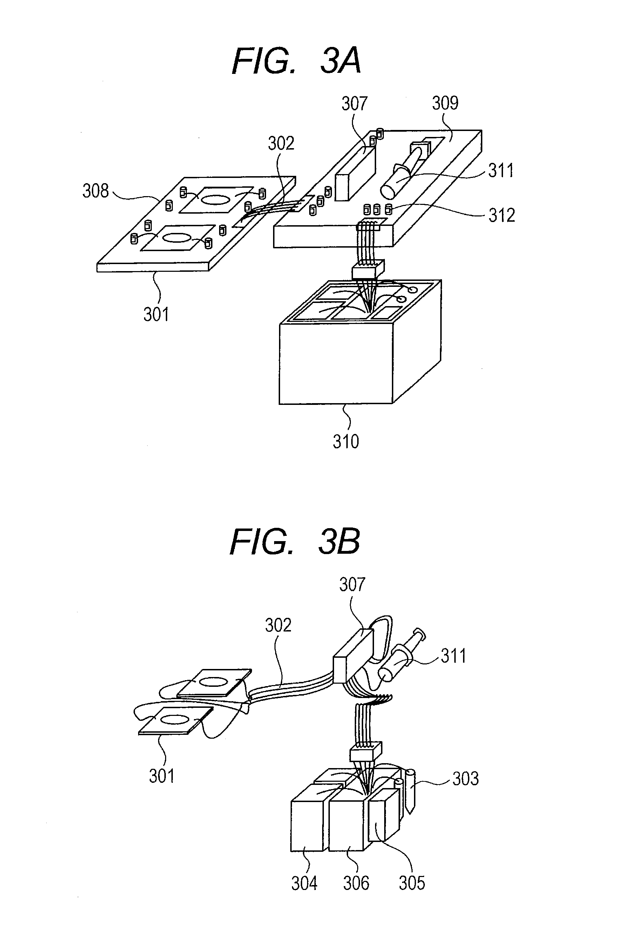

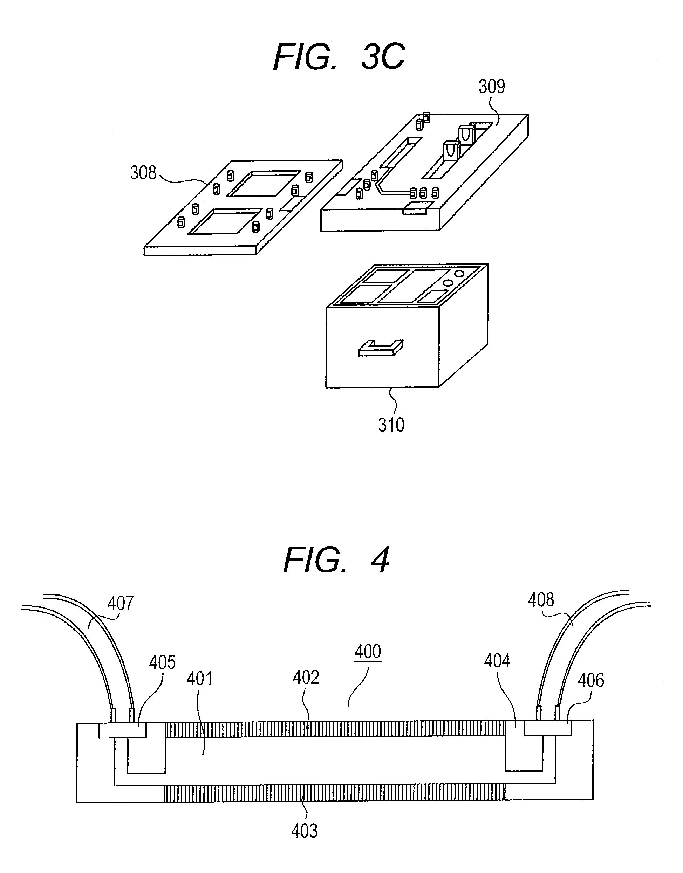

FIG. 1 illustrates an overall configuration of an automatic culture equipment including a flow channel. FIG. 2 illustrates an overall configuration of the automatic culture equipment before providing the flow channel. FIGS. 3A, 3B, and 3C illustrate an overall image of the flow channel which is provided in the automatic culture equipment, a base set that holds a part related to a culture process in the flow channel, and a base set that holds a part of the flow channel related to a culture process, respectively. The flow channel illustrated herein is, for example, a flow channel when two cartridge type closed culture vessels are cultured.

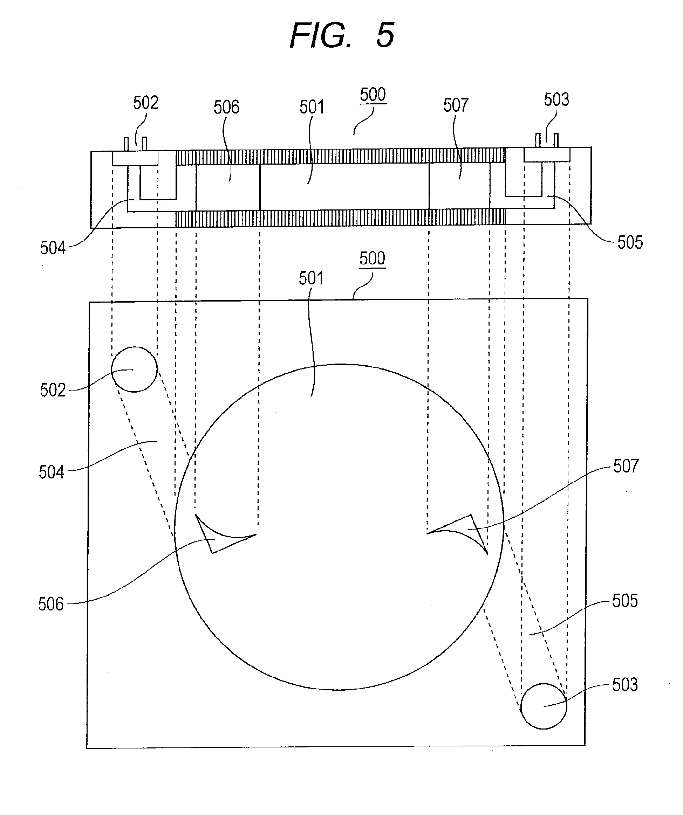

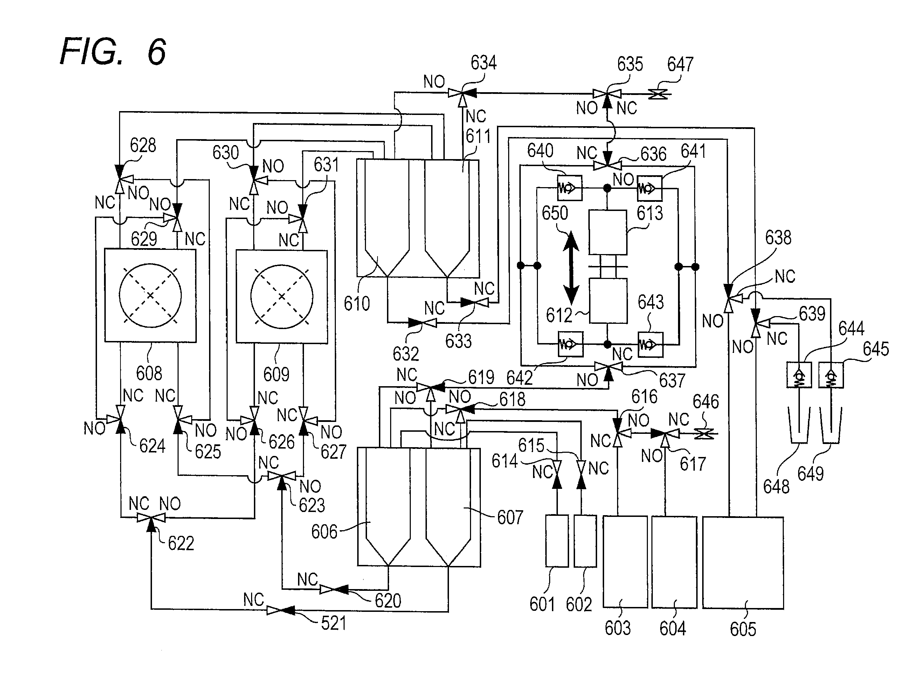

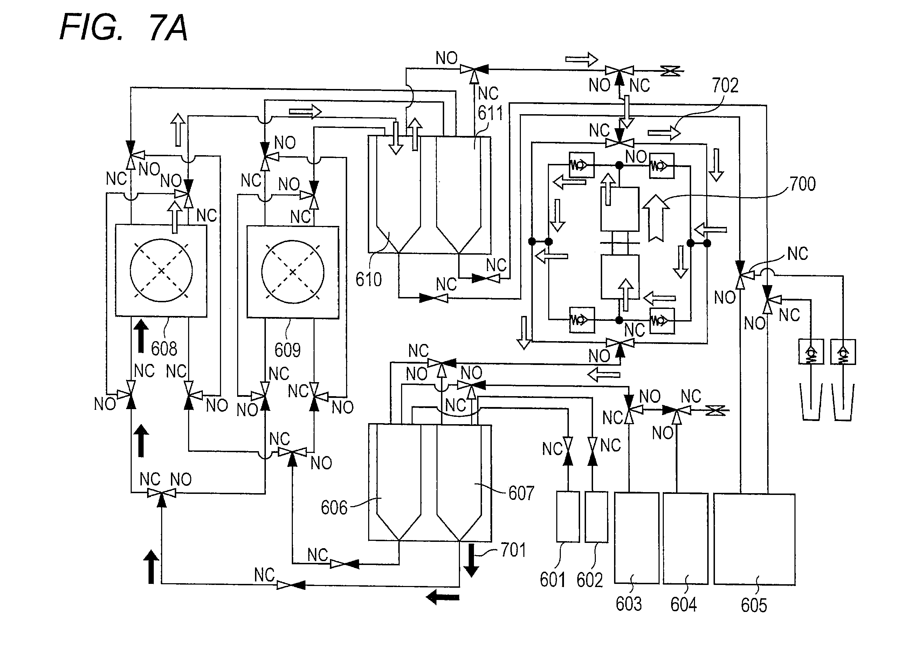

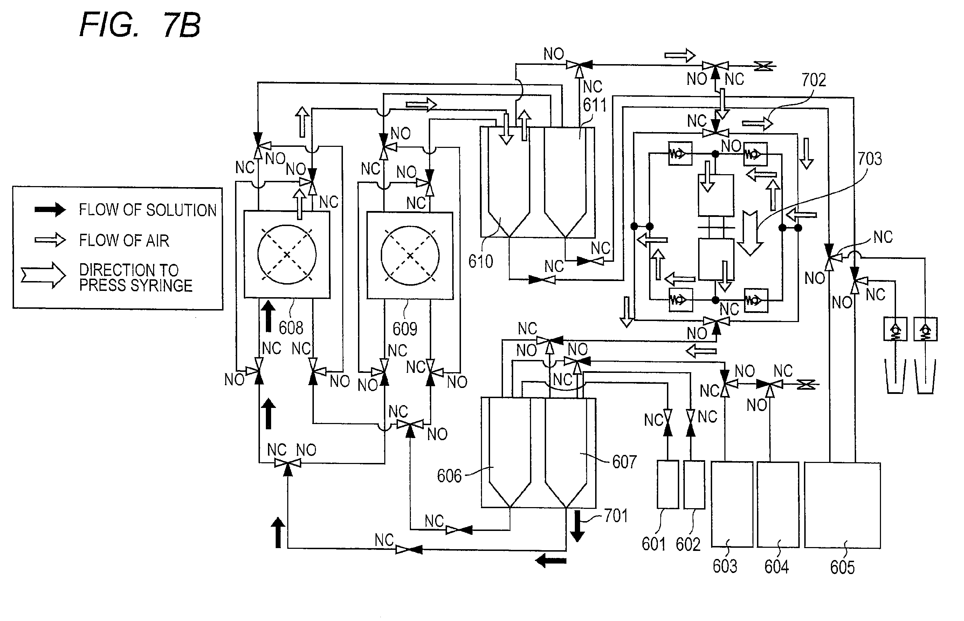

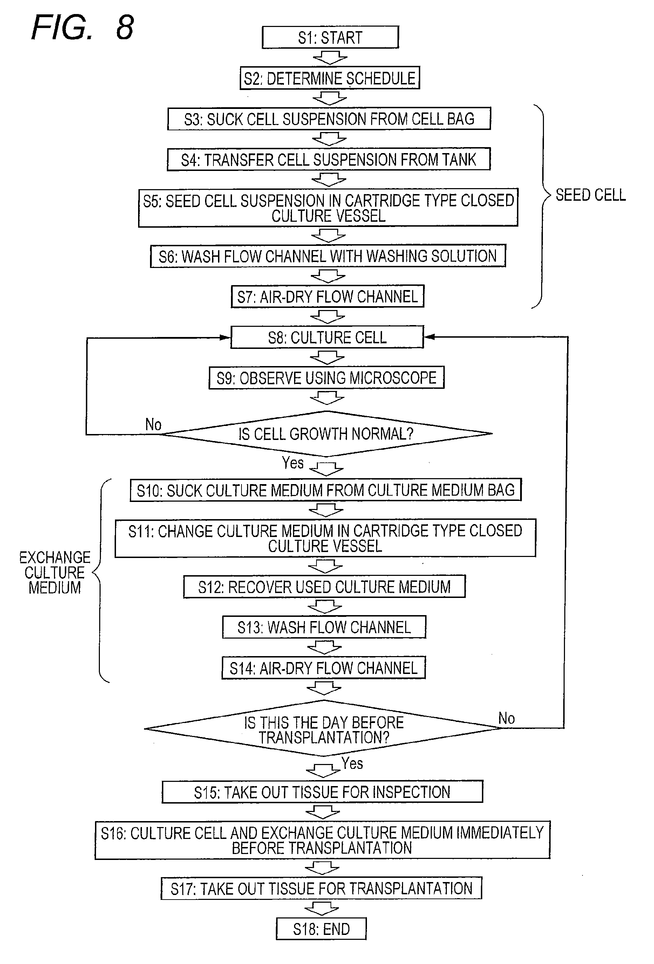

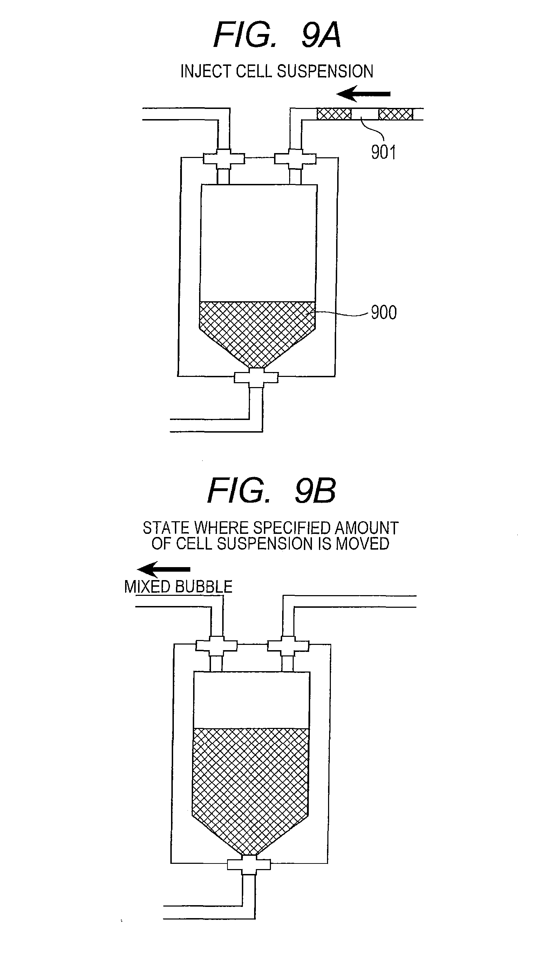

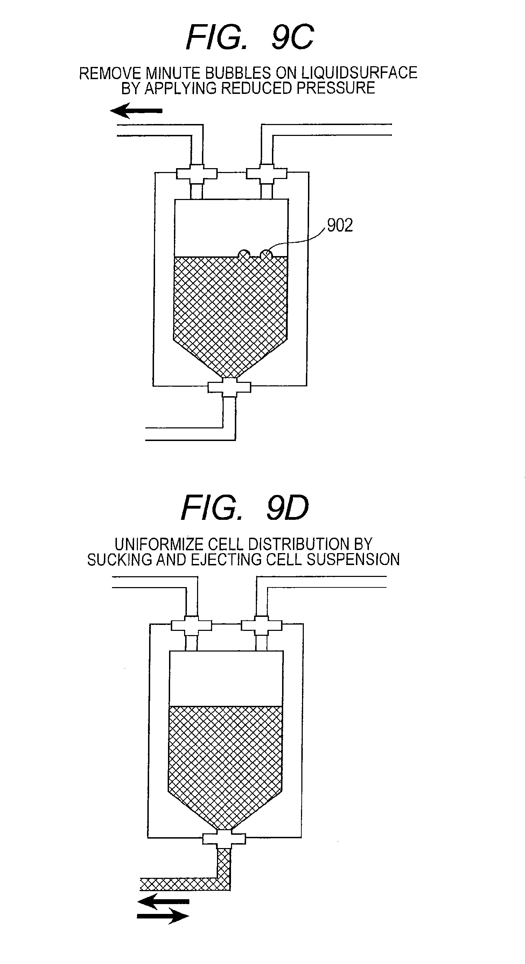

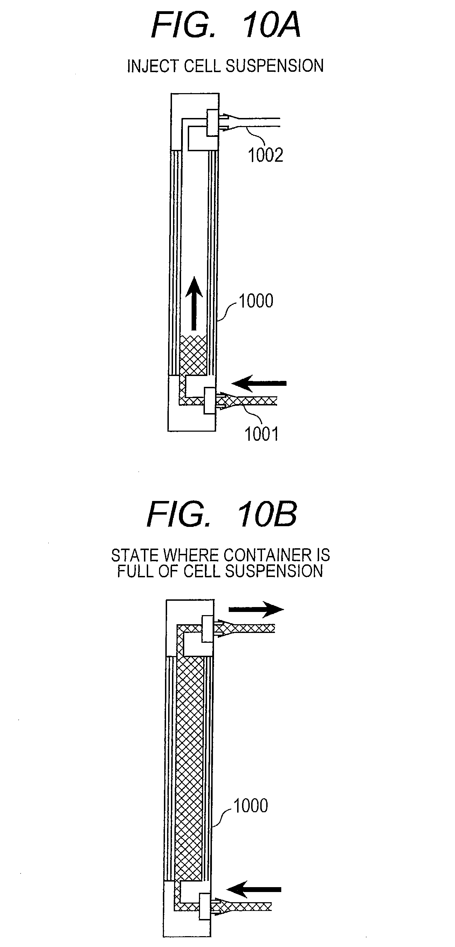

FIG. 4 illustrates a configuration of a cartridge type closed culture vessel according to the first embodiment. FIG. 5 illustrates a state where a diffusing machine, which may diffuse the cells when cell suspension flows in to quickly uniformly seed the cells in a culture space, is provided in the culture space of the cartridge type closed culture vessel, according to a modification embodiment of the first embodiment. FIG. 6 illustrates an overall flow channel when the two cartridge type closed culture vessels are cultured. FIGS. 7A and 7B illustrate movements of a syringe when the solution is flowed from the tank to the cartridge type closed culture vessel, as an example of a flow of a solution and an air that uses the overall view of the flow channel illustrated in FIG. 6. FIG. 8 illustrates a series of protocols that culture the cell using the automatic culture equipment of the present embodiment. FIGS. 9A to 9E illustrate overviews of a protocol which is performed in the tank of the flow channel. FIGS. 10A to 10D illustrate overviews of a protocol which is performed by the flow channel in the cartridge type closed culture vessel.

Referring to FIGS. 1 and 2, basic components of an automatic culture equipment according to the first embodiment will be described. The automatic culture equipment includes cell cultivation rooms 101 and 201, fridges 102 and 202 in which culture medium is stored, controllers 103 and 203, and cleaned air circulating units 104 and 204 that circulates clean internal air. Cell cultivation room doors 105 and 205 and fridge room doors 106 and 206 are provided so that the doors are opened to access the inside of the automatic culture equipment. In the cell cultivation rooms 101 and 201, when cells are cultured, a cartridge type closed culture vessel 107 and a culture vessel base 108 are provided in culture vessel driving units 109 and 209. When culture medium is changed, the cartridge type closed culture vessel 107, the culture vessel base 108, and the culture vessel driving units 109 and 209 rotate as one body by a rotation controller that includes rotation mechanisms 110 and 210 and motors 111 and 211 to erect the cartridge type closed culture vessels 107 and 108 in an approximately perpendicular or vertical direction. In the flow channel, an electronic valve 112, a motor 113, a tank 114, a flow channel base 115, and flow channel driving units 116 and 216 which configure a valve unit are provided to allow various solutions to be flowed.

When the cells are cultivated, the inside of the cell cultivation rooms 101 and 201 is maintained at, for example, a temperature of 37°C, a CO₂ concentration of 5%, and a humidity of 100%. Therefore, a heater 117 which is a heating unit, a temperature sensor 118, a CO₂ supplying mechanism 119, a CO₂ sensor 120, a humidity generating mechanism 121, and a humidity sensor 122 are provided. In order to uniformize the internal environment, the internal air is circulated by a fan 123. Since the culture medium is stored in the fridges 102 and 202, the temperature of the fridges is maintained at 4°C. The culture medium is maintained or kept in the culture vessel and both are set on the culture medium base 124. The culture vessel base 108, the flow channel base 115, and the culture medium base 124 are called as a base 125. Further, the culture vessel driving units 109 and 209 and the flow channel driving units 116 and 216 are referred to as driving units 126 and 226.

A seal 127 is provided between the cell cultivation rooms 101 and 201 and the fridges 102 and 202 to prevent the heat movement between the two spaces. In the clean air circulating units 104 and 204, a cleaning filter such as an HEPA filter (high efficiency particulate air filter) is used to maintain the cleanliness of the cell cultivation rooms 101 and 201. Further, a microscope 128 is used to capture an image of cells which is cultured in the cartridge type closed culture vessel 107 and evaluate a growth situation or state of the cells. In addition, a culture medium component analyzing equipment is connected to the outside, an used culture condition medium obtained as waste solution at the time of changing the culture medium is recovered, and an amount of culture medium components such as glucose or lactic acid, which reflects a growth situation of cells, is measured.

The controllers 103 and 203 include a display unit that displays information such as an internal temperature, a CO₂ concentration, or a humidity, an input unit that performs the manipulation related to a control of the automatic culture equipment, and a communication unit that accesses an external equipment such as a recording device. In order to maintain the cleanness of the automatic culture equipment, the controller has a function that sterilizes or disinfects the inside whenever the culture processes are completed. That is, when the sterilization is performed, the inside of the automatic culture equipment is sterilized by sterilization gas such as ethylene oxide gas, hydrogen peroxide gas, or ozone gas. In this case, materials of the automatic culture equipment have a tolerance against the used sterilization gas and establish a safety without leaking the sterilization gas to the outside of the automatic culture equipment. If the disinfection is performed instead of the sterilization, the disinfection is performed by spraying and/or wiping with a disinfectant such as ethanol. The internal shape preferably has less unevenness in order to easily wipe the inside.

Continuously, the flow channel of the automatic culture equipment of the present embodiment will be described. FIG. 3A illustrates an overall image of the flow channel which is provided in the automatic culture equipment. The flow channel is provided in the automatic culture equipment illustrated in FIG. 2. FIG. 3B illustrates a part of the flow channel which is related to the culture process. FIG. 3C illustrates a base that holds a part of the flow channel, which is related to the culture process, illustrated in FIG. 3B. If the components illustrated in FIGS. 3B and 3C are integrated, a state illustrated in FIG. 3A is obtained.

A configuration of the part related to the culture process mainly includes a cartridge type closed culture vessel 301, a flow channel 302, a cell bag 303 in which a cell suspension is maintained, a culture medium bag 304 in which culture medium is maintained, a cleaning bag 305 in which washing solution is maintained, a waste solution bag 306 in which waste solution is received, and a tank 307.
As described above, a base includes a culture vessel base 308, a flow channel base 309, and a culture medium base 310. Further, a fluid flow controlling mechanism section includes a syringe 311 that changes pressure in the flow channel to flow various solutions and an electronic valve 312 that configures a valve unit.

A detailed configuration of the flow channel will be described with reference to FIG. 6. The flow channel is provided together with a base including a flow channel conveying unit under an environment having a high cleanliness (for example, in the CPC). A cell isolation processing is performed in advance and cell suspension which is in a cultivable state is moved into the cell bag 303 of the flow channel in a safety cabinet. The inside of the safety cabinet is maintained with a very high cleanliness. Therefore, there is no possibility that the inside of the flow channel is contaminated during the operation. Further, the flow channel has a closed system and is configured so that bacteria are not invaded from the outside. Even though slight bacteria are present in the cell cultivation room in the automatic culture equipment, the inside of the flow channel may be maintained to have a high cleanliness from the injection process of the cell suspension to the completion of a cultivation process.

In FIG. 4, an example of the cartridge type closed culture vessel according to the present embodiment is illustrated. In this example, a cartridge type culture vessel 400 has a rectangular parallelepiped shape as a whole, but may have any shape such as a cylindrical shape. In the cartridge type closed culture vessel 400, a fermenter 401 which is a culture space has a cylindrical shape and the top surface and the bottom surface of the cylindrical shape are formed by gas- permeable films 402 and 403 through which gas is permeable from the outside. The gas permeable films 402 and 403 are fixed to a cartridge type closed culture vessel member 404 by an ultrasonic melding unit, for example. A material of the gas-permeable films 402 and 403 is, for example, polycarbonate or silicon polycarbonate. The gas-permeable films 402 and 403 have a lower gas exchangeability than that of a general open culture vessel. However, when the cell is cultured, oxygen or carbon dioxide flows in or out between the inside and the outside through the gas-permeable films. Further, even when an undesirable matter for culture such as bacteria is attached on the outside of the cartridge type culture vessel, the undesirable matter does not enter the inside the gas-permeable films 402 and 403 or the cartridge type closed culture vessel member 404.

Connector units 405 and 406 are attached to the cartridge type closed culture vessel by an adhesive. The flow channels 407 and 408 are coupled to the connector units 405 and 406. Cultivation starts using a connector unit which is sterilized in a connected state and the flow channel is connected at all times until the cultivation is completed. Therefore, a possibility that bacteria are invaded from the outside is significantly lowered. In this example, the cartridge type closed culture vessel 400 having a single layered fermenter 401 is described. However, an intermediate film that has a hole to allow the culture medium to be circulated is provided inside the fermenter 401 and the fermenter 401 may have a two layered structure with a distance therebetween. In this case, the cells are cultured on an upper layer and a lower layer, respectively and the cells may be spatially separated. For example, in case of corneal epithelial regeneration, human derived corneal epithelial stem cells are cultured on the upper layer and mouse derived fibroblast cells are cultured on the lower layer so that the cultivation may be performed by spatially separating different specie derived cells.

FIG. 5 is a modification embodiment of the embodiment of the vessel illustrated in FIG. 4, and illustrates a vessel including a diffusing apparatus, which diffuses the cells when cell suspension flows in to speed up to uniformly seed the cell in a culture space, in the culture space of the cartridge type closed culture vessel. FIG. 5 illustrates a cross-section view of a cartridge type culture vessel 500 and a top view of the cartridge type culture vessel 500. The apparatus that diffuses the cells is mounted therein. The cartridge type culture vessel includes a culture space 501 and connector units 502 and 503. Flow channels 504 and 505 are provided between the culture space 501 and the connector units 502 and 503. Diffusing machines 506 and 507 are provided at a border of the flow channels 504 and 505 and the culture space 501. The diffusing apparatus 506 and 507 are disposed at a position which helps to divide the flow of a cell suspension into two directions and uniformly distribute cells contained in the cell suspension on a culture surface when the cell suspension flows in the culture space 501 from the flow channels 504 and 505. Further, even though the regenerated tissue is manufactured on the culture surface, the diffusing apparatus 506 and 507 have a size which does not interrupt the cultivation so that the regenerated tissue maintains a size required to be used for the transplantation. A shape of the diffusing machine may satisfy the above-mentioned condition and the shape illustrated in FIG. 5 is an example thereof.

An example of the flow channel according to the present embodiment, which may actually culture the cells, using the above-mentioned configuration is illustrated in FIG. 6. In FIG. 6, two cartridge type closed culture vessels 608 and 609 are utilized. Further, as a model, a corneal generation is performed and the fermenters of the cartridge type closed culture vessels 608 and 609 are composed of an upper layer and a lower layer and different cells are cultured on every layers.

As illustrated in FIG. 6, cell bags 601 and 602, a culture medium bag 603, a washing solution bag 604, a waste solution bag 605, tanks 606, 607, 610, and 611, the cartridge type closed culture vessels 608 and 609, syringes 612 and 613, electronic valves 614 to 639, check valves or clack valves 640 to 643 for gas, check valves 644 and 645 for liquid, air filters 646 and 647, and culture medium collection or recovery vessels 648 and 649 are provided. The electronic valves 614 to 639 that configure a valve unit control the flow channel to be open/closed. Basically, two flow channels pass through the electronic valves 614 to 639. If the flow channels are not electrically conducted with the electronic valve, one of the flow channels is open at all times and the other one is closed at all times. If the flow channels are electrically conducted with the electronic valves, the one of the flow channels is closed at all times and the other one is open at all times.

In FIG. 6, among the flow channels that pass through the electronic valves 614 to 639 which are valve units, flow channels denoted by NC (normally closed) are closed when the flow channels are not electrically conducted with the electronic valves. In contrast, flow channels denoted by NO (normally open) are open when the flow channels are not electrically conducted with the electronic valves. The check valves 640 to 645 allow the gas or the liquid to flow only in one direction, but not in a reverse direction. The air filters 646 and 647 appropriately aseptically suck or eject an air from the outside of the flow channel, if necessary. By doing this, the pressure in the flow channel is controlled. The culture medium collection vessels 648 and 649 recover an old culture medium obtained when the culture medium is changed. A culture medium component analyzing device, which is prepared separately from the automatic culture equipment, is used to measure an amount of substance such as glucose or lactic acid, which reflects a growth status of cells, to be used to determine the growth status of the cells. The electronic valves are appropriately open/closed by the flow channels illustrated in FIG. 6 and the inside of the flow channels are appropriately changed by applying a reduced pressure or an elevated pressure by the operation of the syringe to transfer the solution.

For example, open/close operation of the electronic valves and flows of the solution and the air when the solution is flowed onto one of the layers of the cartridge type closed culture vessel 608 from the tank 607 in FIG. 6 are illustrated in FIGS. 7A and 7B. In the electrically conductive state, the electronic valves at NO sides are open and the electronic valves at NC sides are closed. In order to flow the solution, first, the electronic valves 621, 622, 624, and 629 are electrically conductive. Then, the electronic valves open the flow channels denoted by NC and close the flow channels denoted by NO in FIG. 6. In this state, the syringes 612 and 613 operate in a direction of an arrow 650. The syringes 612 and 613 can move to be synchronized with each other. When the syringe 612 transfers or supplies gas, the syringe 613 absorbs gas. In contrast, when the syringe 613 transfers gas, the syringe 612 absorbs gas. Further, the amount of the transferred gas is equal to the amount of the absorbed gas in the respective operations. The two syringes 612 and 613 repeatedly transfer and absorb the gas to continuously transfer the liquid. The liquid in the flow channel is stretched from one side and the liquid is extruded from the other side and two forces are combined to efficiently flow the liquid.

FIG. 7A illustrates flows of the solution and the air when the syringes 612 and 613 are driven in the direction of an arrow 700, the syringe 612 absorbs gas, and the syringe 613 transfers gas. The directions where the solution and the air proceed are represented by a black arrow 701 and a white arrow 702, respectively. The check valves 640 to 643, which are disposed around the syringes, allows the air in one direction to flow, but not in the reverse direction. By the operation of the check valves, the flow of the air is limited so that the solution is transferred as illustrated in the drawing. In the meantime, FIG. 7B illustrates flows of the solution and the air when the syringes 612 and 613 are driven in the direction of an arrow 703 by the operation of the check valves 640 to 643, the syringe 612 transfers gas, and the syringe 613 absorbs gas. The operations illustrated in FIGS. 7A and 7B are repeated and the syringes repeatedly transfer and absorb the gas until a specified amount of solution moves onto one of the layers of the cartridge type closed culture vessel 608 from the tank 607. Other solution may also be transferred by opening/closing appropriate electronic valves and sending the air by the syringe.

A series of culture procedures when the cell is cultured using the automatic culture equipment of the present embodiment with the above-described configuration will be described. FIG. 8 is a flow chart explaining an operation of the automatic culture equipment. Hereinafter, the operation of the automatic culture equipment will be described with reference to the overall view of the automatic culture equipment of FIG. 1, a view of the flow channel of FIG. 6, the operational views in the tank of FIGS. 9A to 9E, and the operational views in the cartridge type closed culture vessel of FIGS. 10A to 10E. Further, if the number of the cartridge type closed culture vessels is increased, the cartridge type closed culture vessel may be arranged in parallel in the flow channel. In addition, as the cultivation procedure in this case, the following manipulations may be sequentially performed on each of the cartridge type closed culture vessels.

### <Step S1: Start>

First, as illustrated in FIG. 8, the automatic culture equipment is activated. The automatic culture equipment is activated when an operator presses a start switch, which is omitted in the drawing, of the manipulation unit in the controller 103 of FIG. 1. Further, at this time, a CO₂ bombe, a flow channel, a culture medium, and cell suspension solution are provided in the automatic culture equipment. On a manipulation screen of the display of the controller 103 which is omitted in the drawing, it is checked whether an internal environment of the automatic culture equipment has appropriate values. For example, as described above, the temperature is 37°C, the CO₂ concentration is 5%, and the humidity is 100%. However, the numerical values are not limited thereto. For example, the temperature may be selected from the range of 0°C to 45°C. In addition, the sterilization using a sterilization gas or disinfection using ethanol is performed to the inside of the equipment in advance by an appropriate manipulation so that the inside of the equipment is in a clean state. In addition, a flow channel which will be used for culture is also sterilized in advance.

### <Step S2: Determine Schedule>

In accordance with a type and amount of cells to be cultured, a schedule of the automatic cultivation performed by the automatic culture equipment is determined. Conditions, such as a date, a frequency, and an amount of liquid when performing the manipulation such as cell seeding, culture medium changing, microscope observing, collection of tissues for inspection, or collection of tissues for transplantation are input from the manipulation unit of the controller 103.

### <Step S3: Suck cell suspension from cell bag>

Steps S3 to S7 of FIG. 8 are the operation of seeding cells. An appropriate electronic valve is open/closed, and then the syringes 612 and 613 operate, and the cell suspension is sucked from the cell bags 601 and 602. In an example of corneal regeneration, the cell suspension is corneal epithelial cells which are suspended in KCM (keratinocyte culture medium) and 3T3 cells which are suspended in the same KCM. As the syringes 612 and 613 are driven, the air in the flow channel is exhausted to the outside of the flow channel through the air filter 647 and the cell suspension is sucked using a reduced pressure state. It takes time until the automatic culture equipment is operated after injecting the cell suspension from the safety cabinet to the cell bags 601 and 602 so that the cell suspension is sunk on the bottom of the cell bags 601 and 602 due to the weight of the cell. Therefore, in order to suck the cell suspension after uniformizing the non-uniform cell suspension, the following manipulations are performed.

That is, before sucking a full amount of cell suspension from the cell bags 601 and 602, two operations of sucking a small amount of cell suspension by switching the electronic valves 614 to 639 and applying a reduced pressure and of injecting the same amount of cell suspension by switching the electronic valves 614 to 639 and applying an elevated pressure are repeated. By doing this, an agitation flow is created in the cell bags 601 and 602. In a stage when the suction and the injection are repeated the sufficient number of times, the sedimentation of the cells are resolved and the distribution is uniformized, the full amount of cell suspension in the cell bags 601 and 602 is sucked and then transferred to the tanks 606 and 607. By performing these operations, it is possible to suck the cell suspension, in which the cells are uniformly distributed, from the cell bags 601 and 602. As a result, the cells which are sunk in the cell bags 601 and 602 are refloated in the solution so that the full amount of cell suspension may be sucked without cell loss. This operation is effective if a small amount of cells is harvested so that the cell loss needs to be avoided as much as possible when a small regenerated tissue is manufactured as in, particularly, the corneal regeneration.

### <Step S4: flow cell suspension from tank>

The cell suspension which is flowed from the cell bags 601 and 602 is temporally received in the tanks 606 and 607.
In FIGS. 9A to 9D, overviews of protocols which are performed in the tanks 606 and 607 are illustrated. First, the cell bags 601 and 602 are received in the fridge 102 whose temperature is, for example, 4°C. Further, the culture medium and the washing solution which is used to change the culture medium are preserved in the fridge at 4°C. Specifically, in the culture medium changing operation performed in S10 to S14, the culture medium is required to be warm at 37°C in advance. Further, the tanks 606 and 607 to be used are common in all operations. When the cells are seeded, the temperature of the cell suspension is required to be warm at 37°C.

Accordingly, as will be described below, the temperature of the cell suspension is raised to 37°C by a heater which is attached around the tanks 606 and 607.
Since the cell suspension is received in the tanks and then heated using the heater, for example, as compared with a case when the cell suspension is heated to 37°C by a heat block in a state where the cell suspension enters the flow channel, it is possible to efficiently raise the temperature. Further, the cell suspension is once received in the tanks 606 and 607 so that it is possible to remove air, which is mixed in the cell suspension, due to some reasons from the cell bags 601 and 502 or the filters 646 and 647.

In a state where a cell suspension 900 is injected as illustrated in FIG. 9A and a specified amount of cell suspension is moved as illustrated in FIG. 9B, when the cell suspension containing bubbles 901 of mixed air reaches the tanks 606 and 607, the cell suspension is collected on the bottom of the tanks 606 and 607 so that the bubbles 901 of the air are removed through the flow channel. Further, if minute bubbles 902 are generated on the liquid surface, the syringes 612 and 613 illustrated in FIG. 6 are driven to reduce a pressure in the tanks 606 and 607 and remove the minute bubbles 902 present on the liquid surface of the cell suspension 900. A condition that a time when the reduced pressure state is maintained is set to a shortest time and even though the bubbles 902 on the liquid surface disappear, the amount of dissolved gas which is ejected to the outside of the liquid is small is set. Therefore, it is possible to transfer various solutions without bubbles to the cartridge type closed culture vessels 608 and 609.

If there are bubbles in the cartridge type closed culture vessels 608 and 609, the cells are gathered due to the surface tension of the bubble on the liquid surface, which becomes a cause of the non-uniformity of the cell distribution. Further, due to the presence of the bubbles, cells which are in contact with the bubbles are dried. As described above, it is important to remove the bubbles.

In the tanks 606 and 607, if necessary, the cell suspension is diluted. After inserting the cell suspension in the tanks 606 and 607, a specified amount of culture mediums is sucked from the culture medium bag 603 to transfer the culture medium to the tanks 606 and 607 to dilute the cell suspension. The culture medium is the KCM (keratinocyte culture medium) in the example of the corneal regeneration. The diluted cell suspension may be seeded and cultured in a plurality of cartridge type closed culture vessels. Further, the cell suspension may be seeded with different concentrations for individual cartridge type closed culture vessels. In this case, since the concentrations of the cell suspensions are varied for individual cartridge type closed culture vessels, the time when the cell is grown to be transplantable regenerated tissues varies. Therefore, when the regenerated tissue is transplanted, it is possible to select and use a regenerated tissue having an optimal growth situation for the transplantation from regenerated tissues having different growth situations.

After diluting the cell suspension as necessary, similarly in the cell bags 601 and 602, the agitation flow is created in the tanks 606 and 607 to uniformize the cell distribution. In other words, before sucking the full amount of cell suspension from the tanks 606 and 607, two operations of sucking a small amount of cell suspension and injecting the same amount of cell suspension by switching the electronic valve and applying a positive pressure are repeated. By doing this, the agitation flow is created in the tanks 606 and 607. For example, the syringe 612 sucks air to create a reduced pressure state so that the solution is stretched. Simultaneously, the syringe 613 ejects the same amount of air to create an elevated pressure state so that the solution is extruded. The solution is stretched from an arbitrary direction and extruded from a reverse direction so that the solution moves in the stretched and extruded states. As compared with a case where only one syringe is used, according to the present embodiment, two syringes are used to efficiently move the solution.

In a step where the suction and injection are repeated the sufficient number of times, the sedimentation of the cell is resolved, and the distribution is uniformized, a full amount of cell suspension in the tanks 606 and 607 is sucked and transferred to the cartridge type closed culture vessels 608 and 609. By performing the operations, it is possible to suck the cell suspension, in which the cells are uniformly distributed, from the tanks 606 and 607. As a result, the cells which are being sunk are refloated in the solution so that the full amount of cell suspension may be sucked without cell loss. Further, the uniform cell suspension may be transferred to the cartridge type closed culture vessels 608 and 609, which helps to uniformly seed the cells in the cartridge type closed culture vessel.

### <Simultaneously agitate cell suspension in cartridge and tank>

As described above, in the tanks 606 and 607 and the cartridge type closed culture vessels 608 and 609, before seeding the cells, the two operations of sucking a small amount of cell suspension and injecting the same amount of cell suspension by switching the electronic valves and applying a positive pressure are repeated and the agitation flow is created therein to uniformize the cell distribution. However, the operations in the tanks 606 and 607 and the cartridge type closed culture vessels 608 and 609 may be simultaneously performed. For example, when the cells are seeded in the cartridge type closed culture vessel 608, the cell suspension which is expected to be seeded in the cartridge type closed culture vessel 609 already enters in the tank 606 or 607. In this state, appropriate valves are open/closed so that the operations of sucking a small amount of cell suspension and injecting the same amount of cell suspension by switching the electronic valves and applying a positive pressure are repeated on both cell suspensions in the cartridge type closed culture vessel 608 and the tank 606 or 607. By simultaneously performing the operations, it is possible to reduce the number of operation processes as compared with the case where the operations are individually performed.

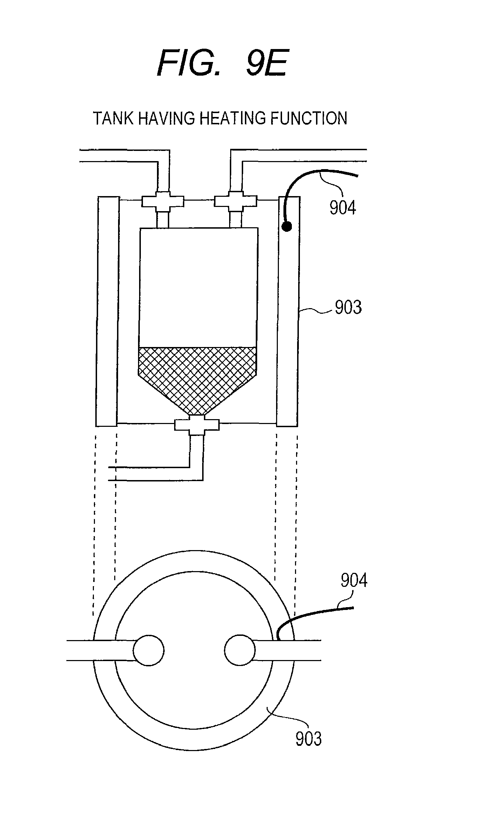

As described, in the tanks 606 and 607, when the culture medium is changed, the culture medium is heated. FIG. 9E is a view illustrating a configuration in which a heater 903 and a temperature sensor 904 are mounted around the tank in order to heat the culture medium. The cell is generally cultured at 37°C. However, a culture medium before being used is stored in the fridge as described above. Therefore, before changing the culture medium, it is required to heat the culture medium at 37°C in advance, which is performed in the tanks 606 and 607 in the present embodiment. The tank is heated by the heater 903 and the heater 903 is controlled so that the temperature measured by the temperature sensor 904 is constantly 37°C. After the tank 904 is heated and a sufficient time to heat the culture medium to 37°C has elapsed, the culture medium is changed using an overheated culture medium.

### <Step S5: flow cell suspension in cartridge type closed culture vessel>

Continuously, in a processing protocol of FIG. 8, the cell suspension flowed from the tanks 606 and 607 is flowed in the cartridge type closed culture vessels 608 and 609.

In FIGS. 10A to 10D, overviews of protocols which are performed in a cartridge type closed culture vessel 1000 are illustrated. First, as illustrated in FIG. 10A, the cell suspension is flowed in a state where the vessel 1000 corresponding to the cartridge type closed culture vessels 608 and 609 is in an upright position. The rotation mechanisms 110 and 210 and the motors 111 and 211 illustrated in FIG. 1 are used to rotate the cartridge type closed culture vessel 1000 to be in an upright position. The cell suspension which is flowed from the tank is injected from a flow channel 1001 which is mounted below the cartridge type closed culture vessel 1000 which is in an upright position. In the meantime, from a flow channel 1002 which is mounted above the cartridge type closed culture vessel 1000 which is in an upright position, the air which is present in the cartridge type closed culture vessel 1000 is extruded.

Accordingly, as illustrated in FIG. 10B, the cell suspension is filled in the vessel 1000 corresponding to the cartridge type closed culture vessels 608 and 609.
Since a reduced pressure is applied in the tanks 606 and 607 as described in step S4, no minute bubble is present. At this timing, since the cell suspension is injected in a state where the cartridge type closed culture vessel 1000 is in an upright position, the cells are sunk due to the weight of the cell and the cells show non-uniform distribution on the culture surface.

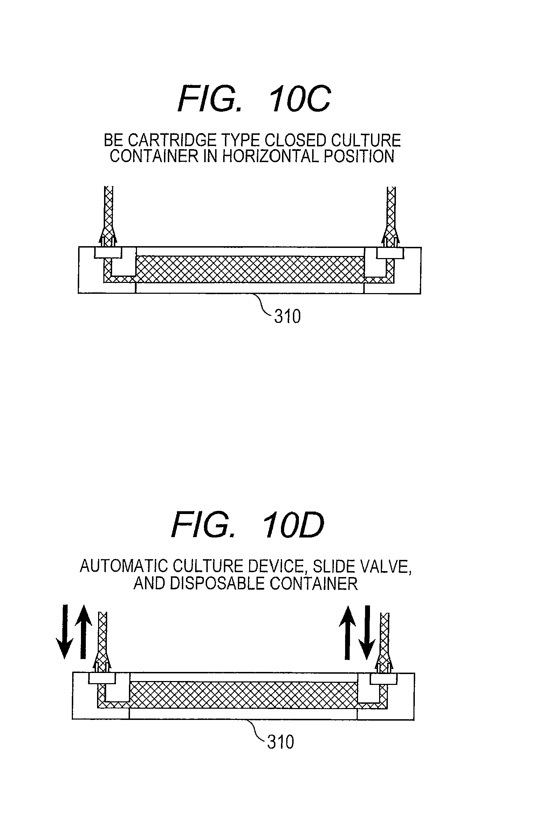

Continuously, as illustrated in FIG. 10C, the cartridge type closed culture vessel 1000 is turned into a horizontal position. As illustrated in FIG. 10D, as performed in the cell bag and the tank, the agitation flow is created in the cartridge type closed culture vessel 1000 to uniformize the cell distribution. That is, two operations of sucking a small amount of cell suspension and injecting the same amount of cell suspension by switching the electronic valves and applying a positive pressure are repeated. By doing this, the agitation flow is created in the cartridge type closed culture vessel 1000. The suction and injection are repeated the sufficient number of times to uniformize the cell distribution. Thereafter, the vessel 1000 corresponding to the cartridge type closed culture vessels 608 and 609 is placed.

### <Step S6: Wash flow channel with washing solution>

Returning to the protocol processing of FIG. 8, the flow channel is washed using a specified amount of washing solution from the washing solution bag 604. As the washing solution, in an example of the corneal regeneration, a pure water or PBS (phosphate buffered saline) solution is used. In case of pure water, even though liquid drops remain in the washed flow channel, nothing remains after vaporizing the moisture. Therefore, pure water is preferable as the washing solution. In the meantime, the PBS solution is also traditionally used as the washing solution. In this case, when the liquid drops remain in the washed flow channel, salt is educed after vaporizing the moisture. Thereafter, if the culture medium is exchanged, the remaining salt is melted in the culture medium to change the composition of the culture medium. For this reason, the PBS solution is not preferable.

In the washing process, first, the washing solution is sucked from the washing solution bag 604 and then flowed to the tanks 606 and 607. The amount of washing solution is preferably the same as the volume of the tank in order to wash the entire tanks 606 and 607. Thereafter, the washing solution is transferred immediately before the cartridge type closed culture vessel. The cleaning solution bypasses the cartridge type closed culture vessel from the electronic valves 624, 625, 626, and 627 immediately before the cartridge type closed culture vessel and is transferred to the electronic valves 628, 629, 630, and 631 immediately after the cartridge type closed culture vessel. Thereafter, the full amount of washing solution is flowed to the tanks 610 and 611. Continuously, the washing solution is moved from the tanks 610 and 611 to the waste solution bag 605. The same manipulation is performed on all tanks and all flow channels. As described above, the flow channels are washed so that it is expect an effect that lowers a probability of biological contamination.

### <Step S7: Air-dry flow channel>

Continuously, after washing the flow channel, the flow channel is air-dried. The entire flow channel is heated using the heater 117 illustrated in FIG. 1 so that superfluous liquid does not remain. Further, the air flows in the flow channel. By doing this, when the culture medium is changed by the continuous manipulations, it is possible to avoid the change in the concentration of the culture medium due to the remaining liquid.

### <Step S8: Culture cell>

The cell is cultured for a specified time in a state where the cartridge type closed culture vessels 608 and 609 are in a horizontal position. For example, in case of the corneal epithelial cells, the placement period is approximately 5 days after seeding the cells. During the cultivation, the internal temperature is maintained at 37°C by the heater 117. The CO₂ concentration is maintained at 5% and the humidity is maintained at 100%. Each of the values is monitored by the temperature sensor 118, the CO₂ sensor 119, and the humidity sensor 120. Further, the air inside the automatic culture equipment is always agitated by a fan 123 so as to always uniformize the distribution of the temperature, the CO₂ and the humidity.

### <Step S9: Observe using microscope>

An image of the cell is obtained using a microscope 128 provided in the automatic culture equipment. Light is appropriately emitted from a light source provided in the automatic culture equipment and the cells are focused by the microscope to capture the image of the cells. If necessary, a fixed point is arbitrarily determined on the culture surface to capture the image. The obtained cell image is stored in a database to be viewed on a display provided outside the automatic culture equipment if necessary. A frequency and time to change the culture medium are adjusted based on information on a growth situation of the cell obtained by observation using the microscope. For example, if the cell is insufficiently attached, the culture medium changing of steps S10 to S14 is not performed but the cell cultivation in step S8 is continued.

### <Step S10: Suck culture medium from culture medium bag>

Steps S10 to S14 are a series of manipulations to change the culture medium in the cartridge type closed culture vessels 608 and 609. The culture medium is generally changed at a frequency of once per several days. The frequency is adjusted in accordance with the growth situation of the cells.

An appropriate electronic valve is open/closed, and then the syringes 612 and 613 operate, and the culture medium is sucked from the culture medium bag 603.
As the syringe is driven, the air in the flow channel is exhausted to the outside of the flow channel through the air filter 647 and the culture medium is sucked using a reduced pressure state. Thereafter, the culture medium is flowed to the tanks 606 and 607. Since the culture medium is cooled at 4°C in the fridge 102, the culture medium is heated to 37°C in the tanks 606 and 607 using the heater.

### <Step S11: Change culture medium in cartridge type closed culture vessel>

The culture medium is flowed from the tanks 606 and 607 to the cartridge type closed culture vessel. In a state where the cartridge type closed culture vessel is in an upright position, the culture medium which is flowed from the tanks 606 and 607 is injected from the flow channel which is mounted below the cartridge type closed culture vessels 608 and 609 which are in an upright position. In the meantime, the used culture medium which remains in the cartridge type closed culture vessels 608 and 609 is extruded from the flow channel which is mounted above the cartridge type closed culture vessels 608 and 609 which are in an upright position. By doing this, new culture medium may be filled in the cartridge type closed culture vessels. In a step where the cartridge type closed culture vessel is full of the new culture medium, the electronic valves 628, 629, 630, and 631, which are disposed near the cartridge type closed culture vessel, are closed. The full amount of old culture mediums is moved to the tanks.

### <Step S12: Recover old culture medium>

The used culture mediums which are moved to the tanks 610 and 611 are moved to the waste solution bag 605. At the time of movement, the electronic valves 638 and 639 operate and some of the used culture mediums are moved to the collection vessels 648 and 649. The check valves 644 and 645 are provided in an ejecting unit of the used culture medium so that the solution flows only in one direction. By doing this, bacteria are prevented from entering from the outside and the inside of the flow channels is prevented from being contaminated. Component of the recovered used culture medium are analyzed by a culture medium component analysis which is separately prepared. For example, an amount of glucose which is used when the cells grow and an amount of discharged lactic acid are measured to find the growth situation of the cells. Further, a mycoplasma test is performed to determine whether the culture medium is biologically contaminated. When the culture medium is contaminated, the cultivation is immediately stopped and the cells are aseptically destroyed by appropriate manipulation so as not to contaminate the place where the automatic culture equipment is provided.

### <Step S13: Washing flow channel>

By the similar method to step S6, a specified amount of washing solution from the washing solution bag 604 is used to wash the flow channel. First, the washing solution is sucked and then transferred to the tanks 606 and 607. The amount of solution is preferably equal to the volume of the tank in order to wash the entire tank. Thereafter, the washing solution is transferred immediately before the cartridge type closed culture vessels 608 and 609. The washing solution bypasses the cartridge type closed culture vessels 608 and 609 from the electronic valves 624, 625, 626, and 627 immediately before the cartridge type closed culture vessel and is transferred to the electronic valves 628, 629, 630, and 631 immediately after the cartridge type closed culture vessel. Thereafter, the full amount of washing solution is flowed to the tanks 610 and 611. Continuously, the washing solution is moved from the tanks 610 and 611 to the waste solution bag 605. The same manipulation is performed on all tanks and all flow channels.

### <Step S14: Air-dry flow channel>

By the similar method to step S7, after washing the flow channel, the flow channel is air-dried. The entire flow channel is heated using the heater so that superfluous liquid does not remain.

### <Step S15: Recover tissue for inspection>

On the day before a scheduled transplantation date, one of the cartridge type closed culture vessels 608 and 609 is taken out for the purpose of inspection. The flow channel which protrudes from the cartridge type closed culture vessel is cut to separate the cartridge type closed culture vessels. The flow channel is blocked using clips at two adjacent locations on the flow channel. The flow channel between the two locations which is blocked by the clips is cut. In the collected cartridge type closed culture vessel, it is tested whether the state of the regenerated tissue therein has a quality enough to perform transplantation. For example, in case of corneal regeneration, it is evaluated whether the cultured tissue has a three layered structure by a histological evaluation. Further, it is evaluated whether the corneal epithelium stem cells are present on a basal layer by the immunohistochemical staining evaluation.

### <Step S16: Culture cell and change culture medium immediately before transplantation>

The cell is cultured by the same manipulation as step S8. Immediately before performing step S17, the culture medium is changed by the same manipulations as steps S10 to S14.

### <Step S17: Take out tissue for transplantation>

As a result of the evaluation by step S15, if it is determined that regenerated tissues suitable for transplantation may be cultured, the tissues for transplantation are recovered to be used for the regenerative medical treatment. Similar to step S15, the flow channel which protrudes from the cartridge type closed culture vessel is cut to separate the cartridge type closed culture vessels. The flow channel is blocked using clips at two adjacent locations on the flow channel. The flow channel between the two locations which is blocked by the clips is cut. Thereafter, the cartridge type closed culture vessel is recovered and the regenerated tissues are conveyed to an operation room where the regenerative medical treatment is performed while maintaining aseptic property and biological quality, to be used for the treatment.

### <Step S18: End>

The flow channel which is used for cultivation is separated.
Continuously, the sterilization using a sterilization gas or disinfection using ethanol is performed to the inside of the equipment by an appropriate manipulation so that the inside of the equipment is in a clean state. Various softwares of the automatic culture equipment are completed and the operation of the automatic culture equipment is completed.

As described above, an example of the embodiment of the present invention has been described with reference to the drawings. However, it is obvious that the present invention is not limited to the embodiments. For example, as a fluid movement controlling mechanism section that moves a fluid in the embodiment, the syringe pump has been described as an example. However, it is also understood that other driving mechanism such as a Peristaltic pump may be used instead of the syringe pump.

According to an appropriate embodiment of the automatic culture equipment with the above configuration, in order to manufacture a uniform regenerated tissue, the cells may be uniformly seeded on the culture surface of the cartridge type closed culture vessel at the time of starting cultivation. Further, the cell suspension, in which cells are uniformly distributed, may be transferred from the cell bag and the tank. As a result, it is possible to seed the cells on the culture surface without the cell loss. In addition, it is possible to remove bubbles which are a cause of the non-uniformity of the cells.

### Industrial Applicability

The present invention is effective as an automatic culture equipment that uses a cartridge-type closed culture vessel to culture cells or tissues by automatic manipulation, and particularly, as an automatic culture equipment which is capable of manufacturing a regenerated tissues which may be used in a regenerative medical treatment.

### Reference Signs List

101, 201 Cell cultivation room
102, 202 Fridge
103, 203 Controller
104, 204 Cleaned air circulating unit
105, 205 Cell cultivation room door
106, 206 Fridge door
107 Cartridge type closed culture vessel
108, 308 Culture vessel base
109, 209 Culture vessel driving unit
110, 210 Rotation mechanism
111, 113, 211 Motor
112, 312, 614 to 639 Electronic valve
114, 307, 606, 606, 610, 611 Tank
115 Flow channel base
116, 216 Flow channel driving unit
117, 901 Heater
118, 902 Temperature sensor
119 CO₂ supplying mechanism
120 CO₂ sensor
121 Humidity generating mechanism
122 Humidity sensor
123 Fan
124, 310 Culture medium base
125 Base
126, 226 Driving unit
127 Seal
128 Microscope
301, 400, 500, 608, 609 Cartridge type closed culture vessel
302, 407, 408 Flow channel
303, 601, 602 Cell bag
304, 603 Culture medium bag
305, 604 Washing solution bag
306, 605 Waste solution bag
309 Flow channel base
311, 612, 613 Syringe
401 Fermenter
402, 403 Gas-permeable film
404 Cartridge type closed culture vessel member
405, 406 Connector unit
501 Culture space
502, 503 Connector unit
504, 505 Flow channel
506, 507 Diffusing apparatus
640 to 643 Check valve for gas
644, 645 Check valve for liquid
646, 647 Air filter
648, 649 Culture medium collection vessel
900 Air bubble
902 Temperature sensor

## Claims

1. An automatic culture equipment that uses a cartridge type closed culture vessel having a culture space therein, the equipment comprising:
a first flow channel which is connected around one end of the cartridge type closed culture vessel and supplies a fluid in the culture space;
a second flow channel which is connected around the other end of the cartridge type closed culture vessel and ejects the fluid in the culture space; and
a fluid flow controlling mechanism section that is connected to the first flow channel and the second flow channel and controls to move the fluid to the cartridge type closed culture vessel;
wherein the fluid movement controlling mechanism section repeatedly supplies and sucks a small amount of cell suspension to the first flow channel in a state where the inside of the cartridge type closed culture vessel is full of the fluid cell suspension to control to create an agitation flow in the cell suspension in the cartridge type closed culture vessel.

2. The automatic culture equipment according to claim 1,
wherein the fluid flow controlling mechanism section includes:
a rotation controller that controls the cartridge type closed culture vessel to select any one of a state in which the cartridge type closed culture vessel is in an approximately upright position so as to dispose the second flow channel at an upper side and the first flow channel at a lower side and a state in which the cartridge type closed culture vessel is in an approximately horizontal position.

3. The automatic culture equipment according to claim 2,
wherein when the cell suspension is flowed in the culture space, the fluid movement controlling mechanism section fills the cell suspension in the cartridge type closed culture vessel in a state in which the cartridge type closed culture vessel is in an approximately upright position by the rotation controller,
after filling the cartridge type closed culture vessel with the cell suspension, controls the rotation controller to maintain the cartridge type closed culture vessel in an approximately horizontal position, and
in a state in which the cartridge type closed culture vessel is in an approximately horizontal position,
repeatedly supplies a small amount of the cell suspension to the first flow channel and repeatedly sucks the cell suspension.

4. The automatic culture equipment according to claim 1,
wherein the fluid flow controlling mechanism section includes:
a first syringe connected through the first flow channel;
a second syringe which is connected through the second flow channel and moves in a synchronized state with the first syringe; and
check valves which are provided between the first flow channel and the first syringe and between the second flow channel and the second syringe to limit the flow in one direction.

5. The automatic culture equipment according to claim 4,
wherein the fluid movement controlling mechanism section operates the first syringe and the second syringe,
allows the first syringe to apply a reduced pressure into the first flow channel and allows the second syringe to apply an elevated pressure into the second flow channel, and changes the pressure from both sides of the cell suspension which fills the cartridge type closed culture vessel to move the cell suspension to the first flow channel side, and
allows the first syringe to apply an elevated pressure into the first flow channel and allows the second syringe to apply a reduced pressure into the second flow channel, and changes the pressure from both sides of the cell suspension which fills the cartridge type closed culture vessel to move the cell suspension to the second flow channel side.

6. The automatic culture equipment according to claim 1,
wherein the cartridge type closed culture vessel includes:
a diffusing apparatus that diffuses the cells in the cell suspension and uniformly seeds the cells in the culture space when the cell suspension flows in the culture space through the first flow channel or the second flow channel.

7. An automatic culture equipment that uses a cartridge type closed culture vessel having a culture space therein, the equipment comprising:
a cell bag in which cell suspension is received;
a culture medium bag in which culture medium is received;
a tank which is disposed between the cartridge type closed culture vessel and the cell bag to temporally reserve the cell suspension;
a first flow channel which is provided above the tank and connects the cell bag, the culture medium bag, and the tank;
a second flow channel that ejects gas in the tank;
a third flow channel that is provided below the tank and connects the cartridge type closed culture vessel and the tank;
a valve unit that opens and closes the first flow channel, the second flow channel, and the third flow channel; and
a fluid flow controlling mechanism section that controls to supply the cell suspension and the culture medium to the tank and dilute the cell suspension,
wherein the fluid movement controlling mechanism section controls to eject bubbles in the cell suspension in the tank to the second flow channel and remove the bubbles from the cell suspension in the tank.

8. The automatic culture equipment according to claim 7, further comprising:
a heating unit that heats the tank and maintains the heated state.

9. The automatic culture equipment according to claim 7,
wherein the fluid flow controlling mechanism section sucks the air from the second flow channel while the first flow channel and the third flow channel are closed by the valve unit to apply a reduced pressure to the tank and maintain the state and removes the bubbles generated on the liquid surface of the cell suspension and then gradually returns the pressure in the tank to a normal pressure.

10. The automatic culture equipment according to claim 7,
wherein the fluid flow controlling mechanism section repeatedly transfers a small amount of the cell suspension collected in the tank when the cell suspension is transferred to the cartridge type closed culture vessel and repeatedly injects the transferred cell suspension in the tank to create an agitation flow in the cell suspension in the tank.

11. The automatic culture equipment according to claim 7,
wherein the fluid movement controlling mechanism section includes:
a first syringe connected to the first flow channel;
a second syringe which is connected through the second flow channel and the third flow channel and moves in a synchronized state with the first flow channel; and
check valves which are provided between the first flow channel and the first syringe and between the second flow channel and the third flow channel and the second syringe to limit the flow in one direction.

12. The automatic culture equipment according to claim 11,
wherein the fluid flow controlling mechanism section controls the valve unit to connect one of the second flow channel and the third flow channel with the second syringe,
operates the first syringe and the second syringe,
allows the first syringe to apply a reduced pressure into the first flow channel and allows the second syringe to apply an elevated pressure into the second flow channel or the third flow channel, and changes the pressure from both sides of the fluid which fills the tank to move the fluid to the first flow channel side, and
operates the first syringe and the second syringe to allow the first syringe to apply an elevated pressure into the first flow channel and allow the second syringe to apply a reduced pressure into the second flow channel or the third flow channel, and changes the pressure from both sides of the fluid which fills the tank to move the fluid to the second flow channel side or the third flow channel side.

13. An automatic culture equipment that uses a cartridge type closed culture vessel having a culture space therein, the equipment comprising:
a cell bag in which the cell suspension is maintained;
a flow channel which is connected to the cell bag; and
a fluid flow controlling mechanism section that controls to move the cell suspension in the cartridge type closed culture vessel through the flow channel,
wherein the fluid flow controlling mechanism section controls to apply a reduced pressure in the flow channel, change the pressure with respect to the cell suspension in the cell bag and move the cell suspension to the flow channel side, and
repeatedly flows a smaller amount of the cell suspension from the cell bag and injects the transferred cell suspension in the cell bag when the cell suspension is transferred, to create an agitation flow in the cell suspension in the cell bag.

14. The automatic culture equipment according to claim 13,
wherein the fluid flow controlling mechanism section includes a syringe which is connected through the flow channel.

15. The automatic culture equipment according to claim 13, further comprising:
a check valve that is provided between the flow channel and the fluid flow controlling mechanism section to limit the flow of the cell suspension in one direction; and a filter that ejects the gas in the flow channel,
wherein the fluid flow controlling mechanism section ejects the gas in the flow channel through the filter to apply a reduced pressure to the flow channel.
